(19) Europäisches Patentamt
European Patent Office
Office européen des brevets

(11) Veröffentlichungsnummer: **0 172 510**
**B1**

(12) # EUROPÄISCHE PATENTSCHRIFT

(45) Veröffentlichungstag der Patentschrift: **26.10.88**

(51) Int. Cl.⁴: **C 07 D 319/20**, A 01 N 47/34

(21) Anmeldenummer: **85110067.7**

(22) Anmeldetag: **10.08.85**

(54) **Benzoylharnstoffe.**

(30) Priorität: **24.08.84 DE 3431221**

(43) Veröffentlichungstag der Anmeldung:
**26.02.86 Patentblatt 86/9**

(45) Bekanntmachung des Hinweises auf die Patenterteilung:
**26.10.88 Patentblatt 88/43**

(84) Benannte Vertragsstaaten:
**AT BE CH DE FR GB IT LI NL**

(56) Entgegenhaltungen:
**EP - A - 0 042 533**
**EP - A - 0 093 976**
**EP - A - 0 093 977**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhövel (DE)**
Erfinder: **Marhold, Albrecht, Dr., Carl-Duisberg-Strasse 329, D-5090 Leverkusen (DE)**
Erfinder: **Becker, Benedikt, Dr., Metzkausener Strasse 14, D-4020 Mettmann (DE)**

**EP 0 172 510 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte 1-Phenyl-3-benzoyl-(thio)harnstoffe, mehrere Verfahren zu ihrer Herstellung und ihre Verwendung als Schädlingsbekämpfungsmittel, insbesondere als Insektizide und Akarizide.

Es ist bereits bekannt, dass bestimmte Benzoylharnstoffe insektizide Eigenschaften besitzen (vgl. z.B. US-PS 4 139 636, US-PS 3 748 356, EP-A 0 042 533, EP-A 0 093 976 und EP-A 0 093 977).

Es wurden die neuen substituierten 1-Phenyl-3-benzoyl-(thio)harnstoffe der allgemeinen Formel (I)

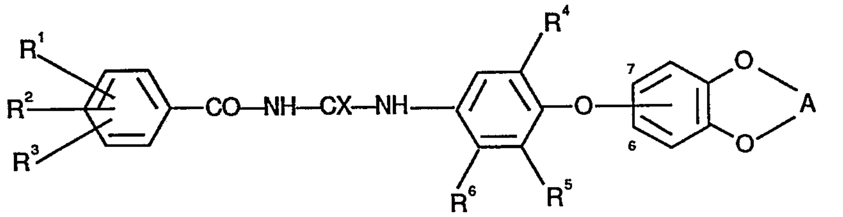

(I)

gefunden,
in welcher
A für die Gruppen $-CF_2-CF_2-$, $-CF_2-CHF-$ oder $-CF_2-CFCl-$ steht,
X für Sauerstoff oder Schwefel steht,
$R^1$ für Halogen oder $C_1-C_4$-Alkyl steht,
$R^2$ und $R^3$ gleich oder verschieden sind und für Wasserstoff, Halogen oder $C_1-C_4$-Alkyl stehen,
$R^4$ und $R^5$ gleich oder verschieden sind und für Wasserstoff, Halogen, $C_1-C_4$-Alkyl oder $C_1-C_4$-Halogenalkyl stehen und
$R^6$ für Wasserstoff steht.

Diese neuen Verbindungen weisen starke biologische, insbesondere insektizide Eigenschaften auf, die ihre Verwendung als Schädlingsbekämpfungsmittel, insbesondere als Insektizide und Akarizide ermöglichen.

Weiterhin wurde gefunden, dass die neuen substituierten 1-Phenyl-3-benzoyl-(thio)harnstoffe der allgemeinen Formel (I) erhalten werden, indem man

a) substituierte Aniline der allgemeinen Formel (II)

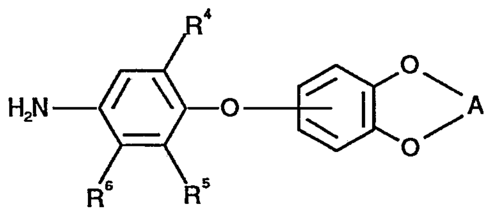

(II)

in welcher
A, $R^4$, $R^5$ und $R^6$ die oben angegebene Bedeutungen haben, mit Benzoyl-iso(thio)cyanaten der allgemeinen Formel (III)

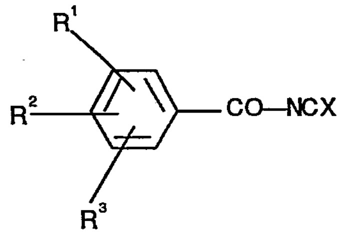

(III)

in welcher
X, $R^1$, $R^2$ und $R^3$ die oben angegebene Bedeutungen haben, gegebenenfalls in Gegenwart eines Verdünnungsmittels umsetzt oder

b) substituierte Phenyl-iso(thio)cyanate der allgemeinen Formel (IV)

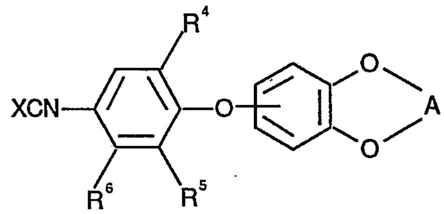

(IV)

in welcher
A, X, $R^4$, $R^5$ und $R^6$ die oben angegebenen Bedeutungen haben, mit Benzoesäureamiden der allgemeinen Formel (V)

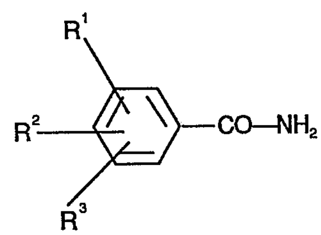

(V)

in welcher
$R^1$, $R^2$ und $R^3$ die oben angegebenen Bedeutungen haben, gegebenenfalls in Gegenwart eines Katalysators und gegebenenfalls in Gegenwart eines Verdünnungsmittels umsetzt.

Die Alkylreste $R^1$, $R^2$, $R^3$, $R^4$, $R^5$ und $R^6$ können gleich oder verschieden sein und bedeuten geradkettiges oder verzweigtes Alkyl mit 1 bis 4 und bevorzugt 1 oder 2 Kohlenstoffatomen. Beispielhaft seien Methyl, Ethyl, n-Propyl, i-Propyl, n-Butyl, i-Butyl, sec.-Butyl, tert.-Butyl genannt.

Besonders bevorzugt wird die Methylgruppe.

Die Halogenalkylreste $R^4$ und $R^5$ können gleich oder verschieden sein und enthalten jeweils im Alkylteil geradkettiges oder verzweigtes Alkyl mit 1 bis 4, insbesondere 1 bis 2 Kohlenstoffatomen

und vorzugsweise 1 bis 6, insbesondere 1 bis 4 Halogenatome, wobei die Halogenatome gleich oder verschieden sind und als Halogenatome vorzugsweise Fluor, Chlor oder Brom, insbesondere Fluor, stehen. Beispielhaft seien Trifluormethyl, Chloridfluormethyl, Trifluorethyl, Chlortrifluorethyl und Tetrafluorethyl genannt. Besonders bevorzugt wird die Trifluormethylgruppe.

A steht vorzugsweise für die $-CF_2-CF_2$-Gruppe.

Halogen bedeutet in der Definition von $R^1$, $R^2$, $R^3$, $R^4$, $R^5$ und $R^6$ Fluor, Chlor, Brom und Jod, vorzugsweise Fluor, Chlor und Brom, insbesondere Fluor und Chlor.

Vorzugsweise ist in den neuen Verbindungen wenigstens einer der Reste $R^1$, $R^2$ und $R^3$ von Wasserstoff verschieden.

Bevorzugt steht X für Sauerstoff.

$R^2$ und $R^6$ bedeuten vorzugsweise Wasserstoff.

$R^1$ steht vorzugsweise in der 2-Position, $R^2$ vorzugsweise in der 4-Position und $R^3$ vorzugsweise in der 6-Position im Phenylring des Benzoylteils.

Der Benzodioxen-Rest ist vorzugsweise in 6- oder 7-Stellung an die an das Sauerstoffatom der Phenoxy-Gruppe gebunden, wobei auch Mischungen der 6- und 7-Isomere vorliegen können.

Die neuen Verbindungen der allgemeinen Formel (I) verfügen über Eigenschaften, die ihre Verwendung als Schädlingsbekämpfungsmittel ermöglichen, insbesondere zeichnen sie sich sowohl durch eine hervorragende insektizide als auch durch eine gute akarizide Wirksamkeit aus. Sie unterscheiden sich hierduch von der bekannten Benzoylharnstoffen, für welche eine insektizide Wirksamkeit bekannt ist.

Die Erfindung betrifft vorzugsweise neue Verbindungen der allgemeinen Formel (I), in welcher
A für die Gruppen $-CF_2-CF_2-$, $-CF_2CH$ oder $-CF_2-CFCl-$ steht,
X für Sauerstoff oder Schwefel, vorzugsweise für Sauerstoff steht,
$R^1$ für Fluor, Chlor, Brom oder Methyl steht,
$R^2$ und $R^3$ gleich oder verschieden sind und für Wasserstoff, Fluor oder Chlor stehen,
$R^4$ und $R^5$ gleich oder verschieden sind und für Wasserstoff, Fluor, Chlor, Methyl oder Trifluormethyl stehen, und
$R^6$ für Wasserstoff steht.

Eine besonders gute Wirksamkeit weisen Verbindungen der allgemeinen Formel (I) auf, in welcher
A für die $-CF_2-CF_2$-Gruppe steht,
X für Sauerstoff oder Schwefel steht,
$R^1$ in der 2-Position ist und für Chlor oder Fluor steht,
$R^2$ für Wasserstoff steht,
$R^3$ in der 6-Position ist und für Wasserstoff, Fluor oder Chlor steht,
$R^4$ und $R^5$ gleich oder verschieden sind und für Wasserstoff, Chlor, Methyl oder Trifluormethyl stehen, und
$R^6$ für Wasserstoff steht.

Verwendet man nach Verfahrensvariante (a) 5-(2,2,3,3-Tetrafluor-benz-1,4-dioxen-6-oxy)-aminobenzol und 2,6-Difluorbenzoylisocyanat als Ausgangsstoffe, so kann der Reaktionsablauf durch das folgende Formelschema wiedergegeben werden:

a)

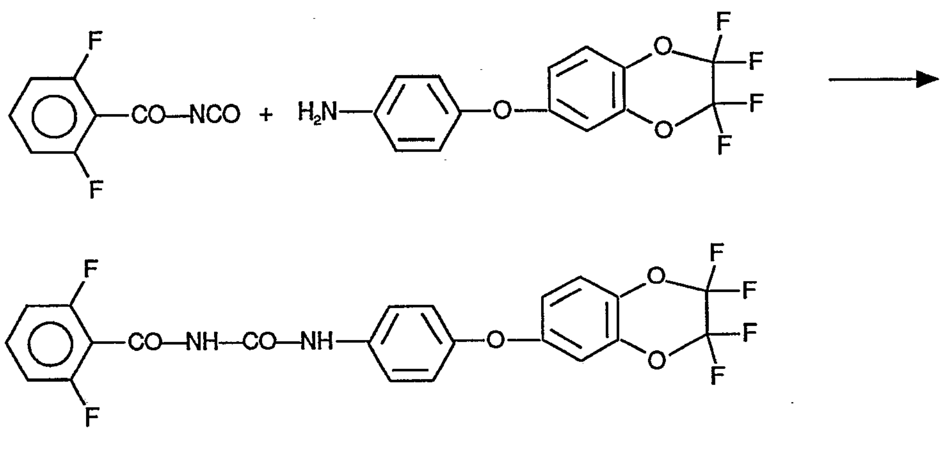

Verwendet man nach Verfahrensvariante (b) 4-(2,2,3,3,-Tetrafluor-benz-1,4-dioxen-6-oxy)-phenylisocyanat und 2,6-Difluor-benzamid als Ausgangsstoffe, so kann der Reaktionsablauf durch das folgende Formelschema wiedergegeben werden:

b)

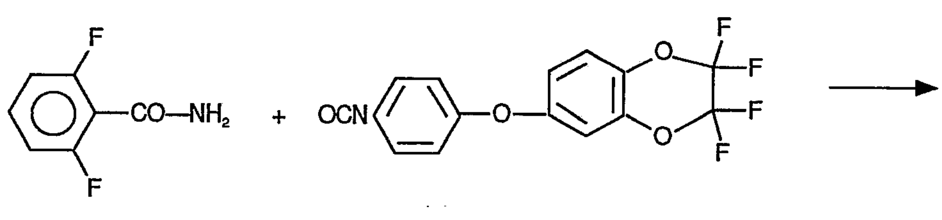

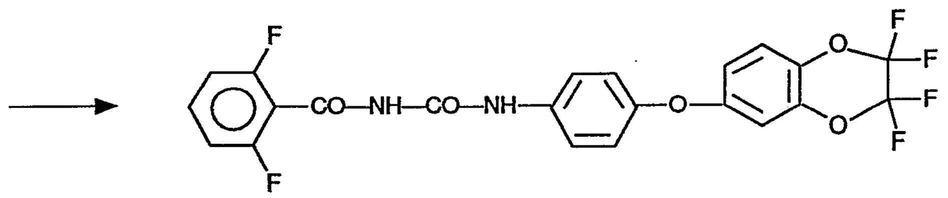

Die Ausgangsverbindungen der allgemeinen Formeln (II) und (IV) sind neu. Ihre Herstellungsverfahren werden weiter unten beschrieben.

Die Ausgangsverbindungen der allgemeinen Formel (III) sind bekannt oder nach allgemein bekannten Methoden erhältlich.

Als Beispiele für die Verbindungen der Formel (III) seien genannt: 2-Fluor-, 2-Chlor-, 2-Brom-, 2-Methyl-, 2,6-Difluor-, 2,6-Dichlor-, 2-Chlor-6-fluor-, 2-Chlor-4-fluor-, 2,4-Difluor-, 2,4-Dichlor-, 2,4,6-Trichlor-, 4-Fluor-, 4-Chlor-, 4-Brom- und 4-Methylbenzoylisocyanat bzw. -benzoylisothiocyanat.

Die Ausgangsverbindungen der allgemeinen Formel (V) sind ebenfalls bekannt oder nach allgemein bekannten Methoden erhältlich.

Als Beispiele für die Verbindungen der Formel (V) seien genannt: 2-Fluor-, 2-Chor-, 2-Brom-, 2-Methyl-, 2,6-Difluor-, 2,6-Dichlor- und 2-Chlor-6-fluorbenzoesäureamid.

Als Verdünnungsmittel kommen bei der Durchführung der Verfahrensvarianten (a) und (b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzung gemäss Verfahrensvariante (b) vorzugsweise tertiäre Amine, wie Triethylamin und 1,4-Diazabicyclo[2,2,2]-octan, sowie organische Zinn-Verbindungen, wie z.B. Dibutylzinndilaurat verwendet werden. Der Zusatz solcher Katalysatoren ist jedoch nicht erforderlich.

Die Reaktionstemperatur kann bei den Verfahrensvarianten (a) und (b) innerhalb eines grösseren Bereiches variiert werden. Im allgemeinen arbeitet man bei der Verfahrensvariante a) zwischen 20 und 180°C, vorzugsweise zwischen 40 und 120°C und bei der Verfahrensvariante b) zwischen 20 und 200°C, vorzugsweise zwischen 60 und 190°C. Die erfindungsgemässen Verfahrensvarianten werden im allgemeinen bei Normaldruck durchgeführt.

Zur Durchführung der erfindungsgemässen Verfahrensvarianten werden die Ausgangsstoffe gewöhnlich in etwa äquimolaren Mengen eingesetzt. Ein Überschuss der einen oder anderen Reaktionskomponenten bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Ausgangsverbindungen der allgemeinen Formeln (II) und (IV) sind neu. Sie können unter der folgenden allgemeinen Formel (VI) zusammengefasst werden:

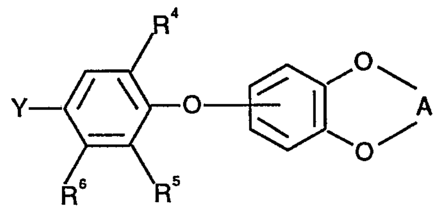 (VI)

in welcher

Y für die Gruppen $NH_2$ oder NCX steht, und

A, X, $R^4$, $R^5$ und $R^6$ die oben angegebenen Bedeutungen haben (wobei hier auch die oben aufgeführten Vorzugsbereiche gelten).

Die Verbindungen der allgemeinen Formel (VI) und Verfahren zu ihrer Herstellung sind Teil der vorliegenden Erfindung.

Die Verbindungen der allgemeinen Formel (VI) werden dadurch erhalten, dass man die Verbindungen der allgemeinen Formel (VII)

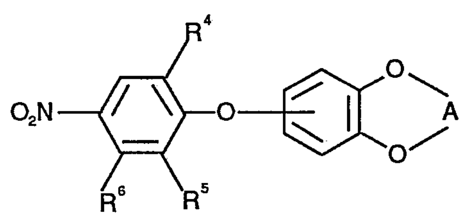 (VII)

in welcher

A, $R^4$, $R^5$ und $R^6$ die oben angegebene Bedeutung haben, katalytisch oder mit Metallen oder Metallsalzen zu den Verbindungen der allgemeinen Formel (II)

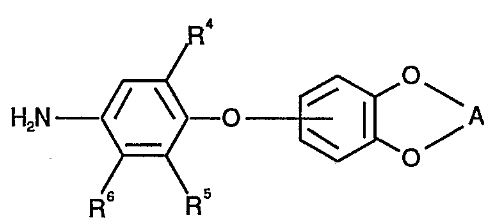 (II)

in welcher
A, $R^4$, $R^5$ und $R^6$ die oben angegebene Bedeutung haben, reduziert und die Verbindungen der allgemeinen Formel (II) isoliert und diese Verbindungen gegebenenfalls mit Phosgen oder Thiophosgen zu den Verbindungen der allgemeinen Formel (IV)

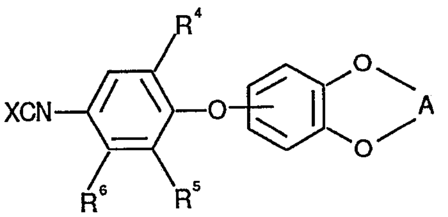 (IV)

in welcher
A, X, $R^4$, $R^5$ und $R^6$ die oben angegebene Bedeutung haben, umsetzt.

Für die katalytische Hydrierung der Nitroverbindungen der allgemeinen Formel (VII) kommen alle üblichen Katalysatoren in Frage, wie Raney-Nickel, Platin, Platinoxid und Palladium, wobei die Katalysatoren auch auf Trägern, z.B. Aktivkohle oder Aluminiumoxid vorliegen können.

Als Lösungsmittel kommen alle bei Hydrierungen üblicherweise eingesetzten Lösungsmittel in Frage, wie Alkohole, z.B. Methanol, Kohlenwasserstoffe, wie Toluol, oder Ether, z.B. Dioxan und Tetrahydrofuran.

Im allgemeinen setzt man erhöhte Wasserstoffdrücke, vorzugsweise zwischen 10 und 80 bar ein. Die Hydrierung wird vorzugsweise bei Temperaturen von 10 bis 100°C, insbesondere 20 bis 80°C durchgeführt. Die Aufarbeitung und Isolierung der Verbindungen der allgemeinen Formel (II) erfolgt in üblicher Weise.

Die Reduktion der Nitroverbindungen der allgemeinen Formel (VII) kann in üblicher Weise auch mit Metallen wie Eisen und Zinn und deren Salzen, vorzugsweise $SnCl_2$, vorgenommen werden. Die Reduktion mit $SnCl_2$ erfolgt vorzugsweise in einem Gemisch aus wässrigen HCl und Dioxan (oder auch wasserfrei in Alkoholen, wie Ethanol) bei Temperaturen zwischen 20 und 100°C. Analog kann auch Eisen in Form von Eisenpulver oder von Eisenspänen verwendet werden, wobei vorzugsweise Temperaturen von 80 bis 100°C eingesetzt werden. Die Aufarbeitung und Isolierung der Verbindungen der allgemeinen Formel (II) erfolgt auch hierbei in üblicher Weise.

Die Verbindungen der allgemeinen Formel (IV) können nach den üblichen Phosgenierungsmethoden leicht aus den Verbindungen der allgemeinen Formel (II) und Phosgen bzw. Thiophosgen erhalten werden. Als Lösungsmittel werden vorzugsweise gegebenenfalls halogenierte aromatische und araliphatische Lösungsmittel wie Chlortoluol, Toluol oder Xylol verwendet.

Die Phosgenierung erfolgt vorzugsweise bei Temperaturen zwischen 0 und 150°C, insbesondere zwischen 0 und 130°C. Die Aufarbeitung und Isolierung der Verbindungen der allemeinen Formel (IV) wird nach allgemein üblichen Methoden vorgenommen.

Die Verbindungen der allgemeinen Formel (VII) sind neu. Diese Verbindungen und ein Verfahren zu ihrer Herstellung sind ebenfalls Teil der vorliegenden Erfindung.

Die Verbindungen der allgemeinen Formel (VII)

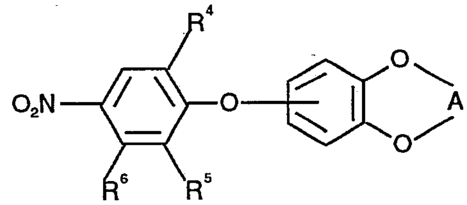 (VII)

in welcher
A, $R^4$, $R^5$ und $R^6$ die oben angegebene Bedeutung haben (wobei hier auch die oben aufgeführten Vorzugsbereiche gelten), werden dadurch erhalten, dass man die Verbindungen der allgemeinen Formel (VIII)

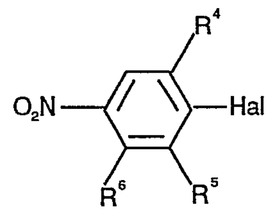 (VIII)

in welcher
Hal für Halogen (vorzugsweise Fluor, Chlor und Brom, insbesondere Chlor oder Brom) steht, mit Verbindungen der allgemeinen Formel (IX)

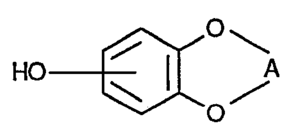 (IX)

in welcher
A die oben angegebene Bedeutung hat, gegebenenfalls in Form ihrer Salze (vorzugsweise Alkali- oder Erdalkalisalze) oder gegebenenfalls in Gegenwart einer Base umsetzt.

Die Umsetzung der Verbindungen der allgemeinen Formeln (VIII) und (IX) zu den Verbindungen der allgemeinen Formel (VII) wird in Gegenwart eines organischen Lösungsmittels durchgeführt, wobei polare organische Lösungsmittel bevorzugt werden, wie Amide, z.B. Dimethylacetamid und Dimethylformamid oder Dimethylsulfoxid.

Die Reaktionstemperaturen liegen bei mehreren Stunden (vorzugsweise 10 bis 20 Stunden) Reaktionsdauer bei 100 bis 180°C, vorzugsweise 110 bis 130°C. Die Reaktanten können in molaren Mengen eingesetzt werden. Ein Überschuss der einen oder anderen Ausgangsverbindungen erbringt jedoch keine nachteiligen Ergebnisse.

Zweckmässigerweise wird eine Base, vorzugsweise ein Alkali- oder Erdalkalihydroxid (in bevorzugt äquivalenter Menge) zugesetzt, wie Natriumhydroxid, Kaliumhydroxid oder Calciumhydroxid. Es ist auch möglich, die Verbindungen der allgemeinen Formel (IX) in Form ihrer Salze, vorzugsweise der Alkali- oder Erdalkalisalze, besonders bevorzugt der Natrium- oder Kaliumsalze einzusetzen. Die Aufarbeitung und Isolierung der Verbindungen der allgemeinen Formel (VII) erfolgt nach allgemein üblichen Methoden.

Die Verbindungen der allgemeinen Formel (IX) sind neu und, ebenso wie ein Verfahren zu ihrer Herstellung, Teil der vorliegenden Erfindung.

Die Verbindungen der allgemeinen Formel (IX) werden dadurch erhalten, dass man die Verbindungen der allgemeinen Formel (X)

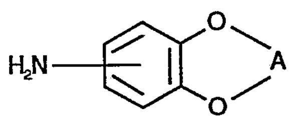

(X)

in welcher

A die oben angegebene Bedeutung hat, diazotiert und das gebildete Diazoniumsalz in saurer Lösung gegebenenfalls in Gegenwart eines Kupfersalzes verkocht und die entstandenen Verbindungen der allgemeinen Formel (IX) in üblicher Weise isoliert.

Die Diazotierung der Verbindungen der allgemeinen Formel (X) erfolgt hierbei nach allgemein üblichen Methoden. Vorzugsweise wird als Verdünnungsmittel eine Mischung aus Schwefelsäure mit Essigsäure und/oder Wasser verwendet. Die Diazotierung wird vorzugsweise mit Natriumnitrit bei Temperaturen von 0 bis 10°C durchgeführt, wobei man zweckmässigerweise 30 bis 60 Minuten bei 20 bis 50°C nachreagieren lässt. Zur Zersetzung des gebildeten Diazoniumsalzes («Verkochen») wird auf 120 bis 180°C in wässriger Schwefelsäure erhitzt, wobei die Schwefelsäurekonzentration bei 40 bis 65% liegen soll.

Zur Erreichung hoher Zersetzungstemperaturen kann der Schwefelsäure bis ca. 30 Gew.-% an Alkalisulfat, vorzugsweise Natriumsulfat zugesetzt werden. Die Reaktion kann durch die Zugabe von Kupfersalzen (wie CuO oder $CuSo_4$) in einer Menge von etwa 5 bis 20 Gew.-%, bezogen auf die Schwefelsäure, erleichtert werden. Das entstehende Hydroxybenzodioxen wird laufend durch Wasserdampfdestillation aus dem Reaktionsgemisch abdestilliert und nach allgemein üblichen Methoden isoliert.

Die Ausgangsverbindungen der allgemeinen Formel (X) sind bekannt oder lassen sich nach bekannten Methoden herstellen (vgl. Europäische Patentschrift Nr. 11 179).

Die neuen Ausgangsstoffe der allgemeinen Formeln (II), (IV), (VII) und (IX) lassen sich in der allgemeinen Formel (XI) zusammenfassen:

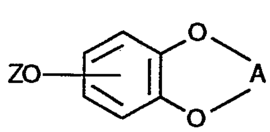

(XI)

in welcher

A für die Gruppen $-CF_2-CF_2-$, $-CF_2-CHF-$ oder $CF_2-CFCl-$ steht,

Z für Wasserstoff oder die Reste

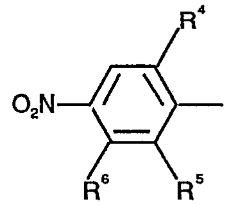

oder

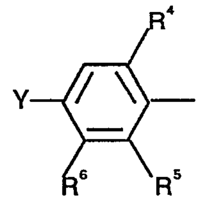

steht, wobei

$R^4$ und $R^5$ gleich oder verschieden sind und für Wasserstoff, Halogen, $C_1-C_4$-Alkyl oder $C_1-C_4$-Halogenalkyl stehen,

$R^6$ für Wasserstoff steht, und

Y für die Gruppen $NH_2$ oder NCX steht, wobei

X Sauerstoff oder Schwefel bedeutet.

Die Wirkstoffe der allgemeinen Formel (I) eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Fannia spp., Calliphora erythrocephala, Lucilia spp., Crysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes app., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstofe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B., nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige

Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.% Wirkstoff, vorzugsweie zwischen 0,0001 und 1 Gew.% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von unerwünschten Schädlingen auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemässen Wirkstoffe geschieht auf diesen Gebieten in bekannter Weise, wie durch äusserliche Anwendung in Form beispielsweise des Tauchens (Dippen), Sprühens (Sprayen) und Aufgiessens (pour-on and spot-on).

Die Wirksamkeit der erfindungsgemässen Verbindungen der allgemeinen Formel (I) soll anhand der folgenden biologischen Beispiele erläutert werden:

Beispiel A

Plutella-Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigten z.B. bei einer Wirkstoffkonzentration von 0,001% die Verbindungen der Beispiele 17, 18 und 27 nach 7 Tagen eine Abtötung von 100%.

Beispiel B

Laphygma-Test

Lösungsmittel: 15 Gewichtsteile Dimethylformamid

Emulgator: 1 Gewichtsteil Alylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigten, z.B. bei einer Wirkstoffkonzentration von 0,0001% die Verbindungen der Beispiele 6, 18 und 25 nach 7 Tagen eine Abtötung von 100%.

Beispiel C

Tetranychus-Test (resistent)

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden, 0% bedeutet, dass keine Spinnmilben abgetötet werden.

Bei diesem Test zeigten z.B. bei einer Wirkstoffkonzentration von 0,1% die Verbindungen der Beispiele 1, 2, 4, 6 bis 9, 11, 15, 18, 19 und 22 bis 27 nach 10 Tagen eine Abtötung von über 95%.

Die Herstellung der erfindungsgemässen Verbindungen der allgemeinen Formel (I) soll anhand der folgenden Beispiele erläutert werden.

Beispiel 1

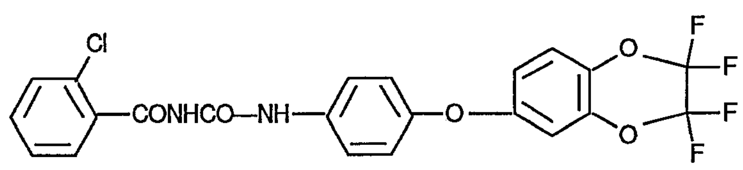

Zu einer Lösung von 3,15 g 4-(2,2,3,3-Tetrafluor-benz-1,4-dioxen-6-oxy)-aminobenzol in 40 ml trockenem Toluol fügt man bei 60°C eine Lösung von 1,82 g 2-Chlorbenzoylisocyanat in 10 ml trokkenem Toluol. Der Ansatz wird ½ Stunde bei 80°C gerührt und danach im Vakuum eingeengt. Nach der Zugabe von etwas Petrolether wird das ausgefallene Produkt abgesaugt. Man erhält 4,4 g des obigen Benzoylharnstoffes (88,5% d.Th.) mit einem Schmelzpunkt von 175°C.

Die folgenden Verbindungen der allgemeinen Formel (I) werden analog Beispiel 1 erhalten:

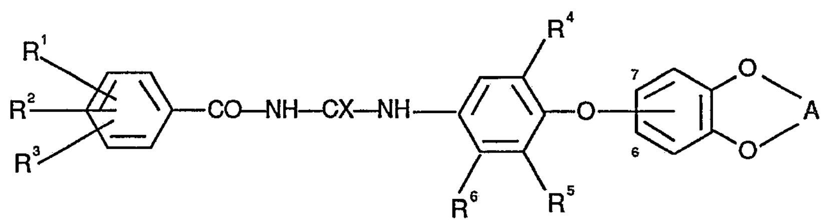

(I)

| Beispiel Nr. | $R^1$ | $R^2$ | $R^3$ | $R^4$ | $R^5$ | $R^6$ | X | Schmp. °C | Ausb.: % d. Th. |
|---|---|---|---|---|---|---|---|---|---|
| | | | | A = $-CF_2-CF_2-$; Benzdioxen-Rest in 6-Stellung | | | | | |
| 2 | 2-F | H | 6-F | H | H | H | O | 188 | 98,5 |
| 3 | 2-Cl | 4-F | H | H | H | H | O | 152 | 97,0 |
| 4 | 2-F | H | 6-F | H | H | H | S | 172 | 86,5 |
| 5 | 2-Cl | H | 6-Cl | H | H | H | S | 163 | 83,0 |
| 6 | 2-F | H | 6-F | Cl | H | H | O | 162 | 80,5 |
| 7 | 2-Cl | h | H | $CH_3$ | H | H | O | 187 | 92,5 |
| 8 | 2-F | H | 6-F | $CH_3$ | H | H | O | 168 | 70,0 |
| 9 | 2-Cl | H | 6-F | $CH_3$ | H | H | O | 116 | 97,5 |
| 10 | 2-Cl | 4-F | H | $CH_3$ | H | H | O | 162 | 90,5 |
| 11 | 2-F | H | 6-F | $CH_3$ | H | H | S | 147 | 66,0 |
| 12 | 2-Cl | H | H | $CF_3$ | h | H | O | 134 | 60,0 |
| 13 | 2-F | H | 6-F | $CF_3$ | H | H | O | 138 | 76,0 |
| 14 | 2-Cl | H | 6-F | $CF_3$ | H | H | O | 153 | 94,5 |
| 15 | 2-F | H | 6-F | $CF_3$ | H | H | S | 165 | 88,0 |
| 16 | 2-Br | H | H | $CF_3$ | H | H | S | 163 | 83,0 |
| 17 | 2-Cl | H | H | Cl | Cl | H | O | 199 | 90,0 |
| 18 | 2-F | H | 6-F | Cl | Cl | H | O | 196 | 90,0 |
| 19 | 2-Cl | H | 6-F | Cl | Cl | H | O | 189-190 | 84,0 |
| 20 | 2-Cl | 4-F | H | Cl | Cl | H | O | 184 | 92,5 |
| 21 | 2-$CH_3$ | H | H | Cl | Cl | H | O | 205 | 99,0 |
| 22 | 2-F | H | 6-F | Cl | Cl | H | S | 141 | 91,5 |
| 23 | 2-Cl | H | 6-F | Cl | Cl | H | S | 198 | 93,5 |
| 24 | 2-Cl | H | H | $CH_3$ | $CH_3$ | H | O | 183 | 85,0 |
| 25 | 2-F | H | 6-F | $CH_3$ | $CH_3$ | H | O | 180-182 | 95,0 |

| Beispiel Nr. | $R^1$ | $R^2$ | $R^3$ | $R^4$ | $R^5$ | $R^6$ | X | Schmp. °C | Ausb.: % d. Th. |
|---|---|---|---|---|---|---|---|---|---|
| | | | A = $-CF_2-CF_2-$; Benzdioxen-Rest in 6-Stellung | | | | | | |
| 26 | 2-Cl | H | 6-F | $Ch_3$ | $CH_3$ | H | O | 181 | 92,5 |
| 27 | 2-F | H | 6-F | $CH_3$ | $Ch_3$ | H | S | 141 | 85,0 |
| 28 | 2-Cl | H | 6-F | $Ch_3$ | $CH_3$ | H | S | 125 | 100,0 |
| 29 | 2-Cl | H | 6-Cl | $CH_3$ | $CH_3$ | h | S | 163 | 81,5 |
| 30 | 2-Cl | H | H | $CF_3$ | $CF_3$ | h | O | 146 | 97,5 |
| 31 | 2-F | H | 6-F | $CF_3$ | $CF_3$ | H | O | 165 | 86,5 |
| 32 | 2-Cl | H | 6-F | $CF_3$ | $CF_3$ | H | O | 172 | 72,5 |
| 33 | 2-Cl | 4-F | H | $CF_3$ | $Cf_3$ | H | O | 151 | 83,0 |
| 34 | 2-F | H | 6-F | $CF_3$ | $CF_3$ | H | S | 147 | 73,5 |

Die Herstellung der Verbindungen der allgemeinen Formel (IX) soll durch die folgenden Beispiele erläutert werden:

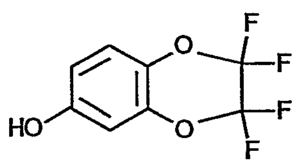

Man legt eine Mischung von 2400 ml Essigsäure und 290 ml konz. Schwefelsäure vor, fügt 316 g 6-Amino-2,2,3,3-tetrafluor-1,4-benzodioxen zu und kühlt auf 5°C ab. Dann wird mit 102 g Natriumnitrit in 360 ml Wasser diazotiert, nach Ende der Zugabe für 30 Minuten bei 20°C und 30 Minuten bei 50°C nachgerührt.

Zur Verkochung des Diazoniumsalzes legt man 2900 ml 50%ige Schwefelsäure mit 220 g Kupfersulfat • $5H_2O$ und 14 g CuO bie Rückfluss vor und tropft die Diazoniumsalzlösung zu. Das entsprechende Phenol wird sofort durch eingeleiteten Wasserdampf abdestilliert. Das Phenol wird aus dem Destillat mit Dichlormethan extrahiert, die organische Phase getrocknet und abdestilliert.

Man erhält 230 g ≙ 72% d.Th an 6-Hydroxy-2,2,3,3-tetrafluorbenzodioxen, Kp: 102–103°C/20 mbar, F=60-62°C.

Beispiele 2 A

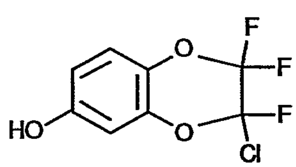

Analog der Vorschrift in Beispiel 1A erhält man aus 6-Amino-3-chlor-2,2,3-trifluor-1,4-benzodioxen 6-Hydroxy-3-chlor-2,2,3-trifluor-1,4-benzodioxen. Kp: 112-5°C/20 mbar.

Beispiel 2B

Aus einer 50:50 Mischung von 6- bzw. 7-Amino-2,2,3-trifluor-1,4-benzodioxen erhält man analog Beispiel 1A eine Mischung von 6- bzw. 7-Hydroxy-2,2,3-trifluor-1,4-benzodioxen.

Kp: 91°C/10 mbar

Die Herstellung der Verbindungen der allgemeinen Formel (VII) soll anhand der folgenden Beispiele erläutert werden:

Beispiel 1B

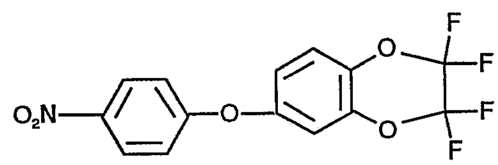

Zu einer Lösung von 3,15 g 2,2,3,3-Tetrafluor-6-hydroxy-benzo-dioxen-1,4 in 200 ml DMSO (= Dimethylsulfoxid) fügt man eine Aufschlämmung von 8,2 g KOH-Pulver in 15 ml DMSO und lässt 1 Stunde bei Raumtemperatur rühren. Zu der Lösung gibt man 18,9 g 4-Nitrochlorbenzol und rührt den Ansatz 14 Stunden bei 110-120°C. Nach dem Abkühlen gibt man 1,5 l Wasser hinzu und extrahiert die Lösung 3 mal mit 250 ml Ethylenchlorid. Die vereinigten Ethylenchloridextrakte werden 2 mal mit Wasser gewaschen und anschliessend getrocknet. Die getrocknete Lösung wird im Vakuum eingeengt und das zurückbleibende Öl mit kaltem Petrolether verrieben, wobei es kristallisiert.

Man erhält 31,7 g obiger Verbindung mit einem Schmelzpunkt von 64°C.

Analog werden die Verbindungen der folgenden Beispiele erhalten:

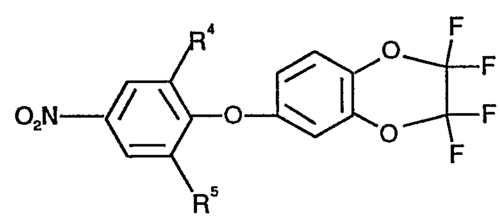

(XII)

| Beispiel Nr. | $R^4$ | $R^5$ | Schmp. 20° C oder $n_D$ | Ausbeute % d. Th. |
|---|---|---|---|---|
| 2B | H | H | 64 | 76 |
| 3B | $CH_3$ | H | 81 | 72,5 |
| 4B | $CF_3$ | H | 1,5015 | 91,5 |
| 5B | Cl | Cl | 61 (1,5515) | 85,5 |
| 6B | $CH_3$ | $CH_3$ | 111 | 78 |
| 7B | $CF_3$ | $CF_3$ | 1,4750 | 89,5 |

Die Herstellung der Verbindungen der allgemeinen Formel (II) soll anhand der folgenden Beispiele erläutert werden:

Beispiel 1C

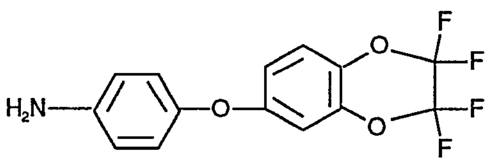

29,3 g 4-(2,2,3,3-Tetrafluor-benz-1,4-dioxen-6-oxy)-nitrobenzol werden in 250 ml Ethanol gelöst. Bei 10–20°C tropft dazu eine Lösung von 92 g $SnCl_2 \cdot 2H_2O$ in 108 ml konz. Salzsäure. Der Ansatz wird 4 Stunden bei 80°C gerührt und dann abgekühlt. Er wird in 1,5 l Eiswasser gerührt, in dem 180 g Natriumhydroxyd gelöst sind. Diese Lösung wird mehrfach mit Ethylenchlorid extrahiert und die vereinigten Extrakte mit Wasser gewaschen. Die Ethylenchloridlösung wird getrocknet und anschliessend das Lösungsmittel im Vakuum verdampft.

Man erhält 24,4 g der Substanz als Öl mit einem Brechungsindex von $n_D^{20}$ 1,5332.

Analog können die Verbindungen der folgenden Beispiele erhalten werden:

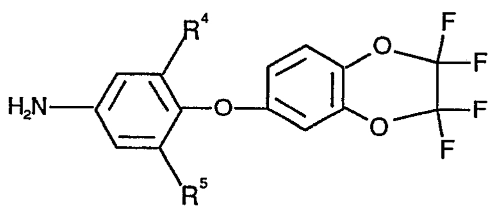

(XIII)

| Beispiel Nr. | $R^4$ | $R^5$ | Schmp. 20° C oder $n_D$ | Ausbeute % d. Th. |
|---|---|---|---|---|
| 2C | H | H | 1,5332 | 86 |
| 3C | $CH_3$ | H | 80 | 76 |
| 4C | $CF_3$ | H | 1,5015 | 92 |
| 5C | Cl | Cl | 136 | 81,5 |
| 6C | $CH_3$ | $CH_3$ | 84 | 95,5 |
| 7C | $CF_3$ | $CF_3$ | 1,4770 | 83 |

**Patentansprüche**

1. Verbindung der allgemeinen Formel (I)

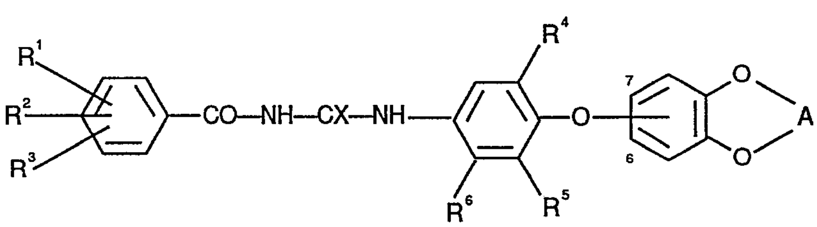

(I)

in welcher
A für die Gruppen $-CF_2-CFCl-$ steht,
X für Sauerstoff oder Schwefel steht,
$R^1$ für Halogen oder $C_1$–$C_4$-Alkyl steht,
$R^2$ und $R^3$ gleich oder verschieden sind und für Wasserstoff, Halogen oder $C_1$–$C_4$-Alkyl stehen,
$R^4$ und $R^5$ gleich oder verschieden sind und für Wasserstoff, Halogen, $C_1$–$C_4$-Alkyl oder $C_1$–$C_4$-Halogenalkyl stehen und
$R^6$ für Wasserstoff steht.

2. Verbindungen gemäss Anspruch 1, in welchen

A für die Gruppen $-CF_2-CF_2-$, $-CF_2-CHF-$ oder $-CF_2-CFCl-$ steht,
X für Sauerstoff oder Schwefel, vorzugsweise für Sauerstoff steht,
$R^1$ für Fluor, Chlor, Brom oder Methyl steht,
$R^2$ und $R^3$ gleich oder verschieden sind und für Wasserstoff, Fluor oder Chlor stehen,
$R^4$ und $R^5$ gleich oder verschieden sind und für Wasserstoff, Fluor, Chlor, Methyl oder Trifluormethyl stehen, und
$R^6$ für Wasserstoff steht.

3. Verfahren zur Herstellung der Verbindungen der allgemeinen Formel (I)

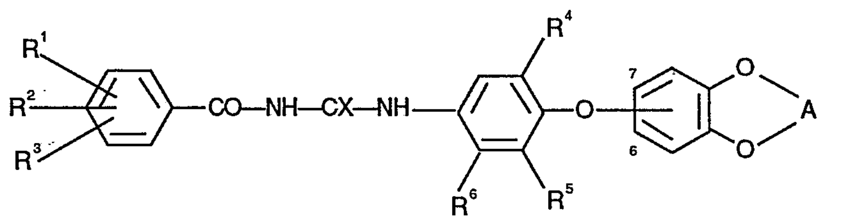

(I)

in welcher
A für die Gruppen $-CF_2-CF_2-$, $-CF_2-CHF-$ oder $-CF_2-CFCl-$ steht,
X für Sauerstoff oder Schwefel steht,
$R^1$ für Halogen oder $C_1-C_4$-Alkyl steht,
$R^2$ und $R^3$ gleich oder verschieden sind und für Wasserstoff, Halogen oder $C_1-C_4$-Alkyl stehen,
$R^4$ und $R^5$ gleich oder verschieden sind und für Wasserstoff, Halogen, $C_1-C_4$-Alkyl oder $C_1-C_4$-Halogenalkyl stehen und
$R^6$ für Wasserstoff steht, dadurch gekennzeichnet, dass man
a) substituierte Aniline der allgemeinen Formel (II)

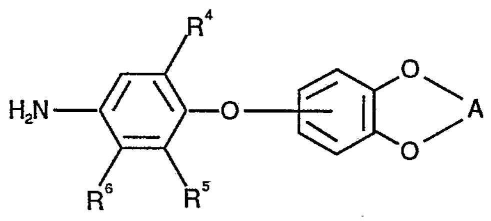

(II)

in welcher
A, $R^4$, $R^5$ und $R^6$ die oben angebenen Bedeutungen haben, mit Benzoyl-iso(thio)cyanaten der allgemeinen Formel (III)

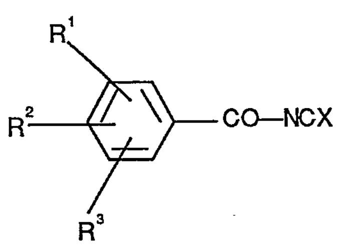

(III)

in welcher
X, $R^1$, $R^2$ und $R^3$ die oben angegebenen Bedeutungen haben, gegebenenfalls in Gegenwart eines Verdünnungsmittels umsetzt oder
b) substituierte Phenyl-iso(thio)cyanate der allgemeinen Formel (IV)

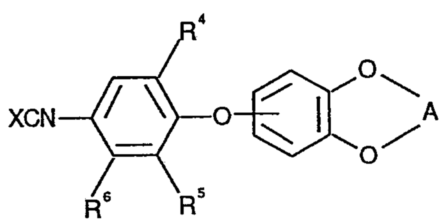

(IV)

in welcher
A, X, $R^4$, $R^5$ und $R^6$ die oben angegebenen Bedeutungen haben, mit Benzosäureamiden der allgemeinen Formel (V)

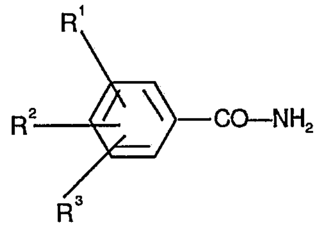

(V)

in welcher
$R^1$, $R^2$ und $R^3$ die oben angegebenen Bedeutungen haben,
gegebenenfalls in Gegenwart eines Katalysators und gegebenenfalls in Gegenwart eines Verdünnungsmittels umsetzt.

4. Schädlingsbekämpfungsmittel, gekennzeichnet durch einen Gehalt an mindestens einer Verbindung der Formel I gemäss den Ansprüchen 1 oder 3.

5. Verwendung von Verbindungen der Formel I gemäss den Ansprüchen 1 oder 3 zur Bekämpfung von Schädlingen, insbesondere Insekten und Milben.

6. Verfahren zur Bekämpfung von Schädlingen, dadurch gekennzeichnet, dass man Verbindungen der Formel I gemäss den Ansprüchen 1 oder 3 auf die Schädlinge, vorzugsweise Insekten oder Milben oder ihren Lebensraum einwirken lässt.

7. Verfahren zur Herstellung von Schädlingsbekämpfungsmitteln, dadurch gekennzeichnet, dass man Verbindungen der Formel I gemäss den Ansprüchen 1 oder 3 mit Streckmitteln und/oder oberflächenaktiven Mitteln vermischt.

8. Verbindungen der allgemeinen Formel (XI)

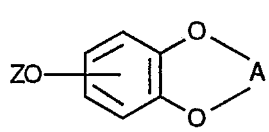

(XI)

in welcher
A für die Gruppen $-CF_2-CF_2-$, $-CF_2-CHF-$ oder $-CF_2-CFCl-$ steht,
Z für Wasserstoff oder die Reste

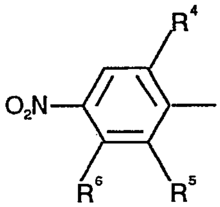

oder

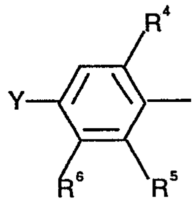

steht, wobei
$R^4$ und $R^5$ gleich oder verschieden sind und für Wasserstoff, Halogen, $C_1$–$C_4$-Alkyl oder $C_1$–$C_4$-Halogenalkyl stehen,
$R^6$ für Wasserstoff steht, und
Y für die Gruppen $NH_2$ oder NCX steht, wobei
X Sauerstoff oder Schwefel bedeutet.

9. Verfahren zur Herstellung der Verbindungen der allgemeinen Formel (XI)

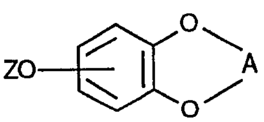

(XI)

in welcher
A für die Gruppen $-CF_2-CF_2-$, $-CF_2CHF-$ oder $-CF_2-CFCl-$ steht,
Z für Wasserstoff oder die Reste

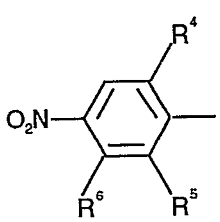

oder

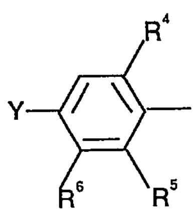

steht, wobei
$R^4$ und $R^5$ gleich oder verschieden sind und für Wasserstoff, Halogen, $C_1$–$C_4$-Alkyl oder $C_1$–$C_4$-Halogenalkyl stehen,
$R^6$ für Wasserstoff steht, und
Y für die Gruppen $NH_2$ oder NCX steht, wobei
X Sauerstoff oder Schwefel bedeutet, dadurch gekennzeichnet, dass man die Verbindungen der allgemeinen Formel (X)

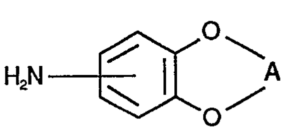

(X)

in welcher
A die oben angegebene Bedeutung hat, diazotiert und das gebildete Diazoniumsalz in saurer Lösung gegebenenfalls in Gegenwart eines Kupfersalzes verkocht und die entstandenen Verbindungen der allgemeinen Formel (IX)

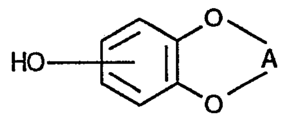

(IX)

in welcher
A die oben angegebene Bedeutung hat, in üblicher Weise isoliert, und gegebenenfalls (zur Herstellung der Verbindungen der allgemeinen Formel (VII)) diese Verbindungen gegebenenfalls in Form ihrer Salze oder gegebenenfalls in Gegenwart einer Base mit Verbindungen der allgemeinen Formel (VIII)

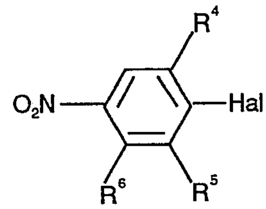

(VIII)

in welcher
Hal für Halogen steht, umsetzt und die erhaltenen Verbindungen der allgemeinen Formel (VII)

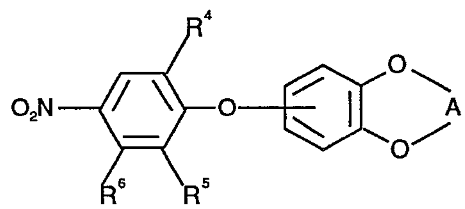

(VII)

in welcher
A, $R^4$, $R^5$ und $R^6$ die oben angegebene Bedeutung haben, in üblicher Weise isoliert und gegebenenfalls (zur Herstellung der Verbindungen der allgemeinen Formel (II)) katalytisch oder mit Metallen oder Metallsalzen reduziert und die erhaltenen Verbindungen der allgemeinen Formel (II)

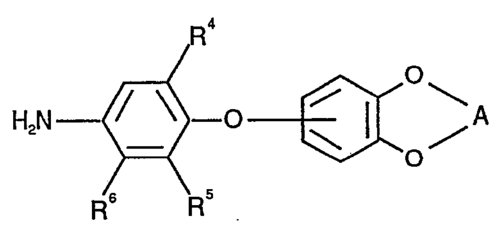

(II)

in welcher
A, $R^4$, $R^5$ und $R^6$ die oben angegebene Bedeutung haben, in üblicher Weise isoliert, und gegebenenfalls (zur Herstellung der Verbindungen der allgemeinen Formel (IV) mit Phosgen bzw. Thiophosgen umsetzt und die erhaltenen Verbindungen der allgemeinen Formel (IV)

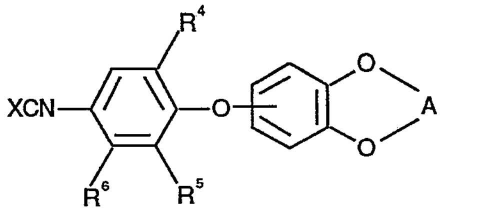

(IV)

in welcher
A, X, $R^4$, $R^5$ und $R^6$ die oben angegebene Bedeutung haben, isoliert.

**Claims**

1. Compounds of the general formula (I)

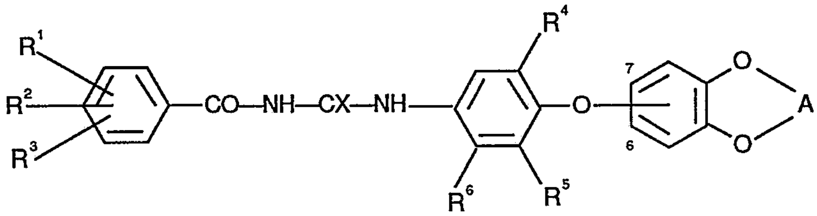

(I)

in which
A represents the $-CF_2-CF_2-$, $-CF_2-CHF-$ or $-CF_2-CFCl-$ group,
X represents oxygen or sulphur,
$R^1$ represents halogen or $C_1-C_4$-alkyl,
$R^2$ and $R^3$ are identical or different and represent hydrogen, halogen or $C_1-C_4$-alkyl,
$R^4$ and $R^5$ are identical or different and represent hydrogen, halogen, $C_1-C_4$-alkyl or $C_1-C_4$-halogenalkyl and
$R^6$ represents hydrogen.

2. Compounds according to Claim 1,
in which
A represents the $-CF_2-CF_2-$, $-CF_2CHF-$ or $-CF_2-CFCl-$ group,
X represents oxygen or sulphur, preferably oxigen,
$R^1$ represents fluorine, chlorine, bromine or methyl,
$R^2$ and $R^3$ are identical or different and represent hydrogen, fluorine or chlorine,
$R^4$ and $R^5$ are identical or different and represent hydrogen, fluorine, chlorine, methyl or trifluormethyl and
$R^6$ represents hydrogen.

3. Process for the preparation of the compounds of the general formula (I)

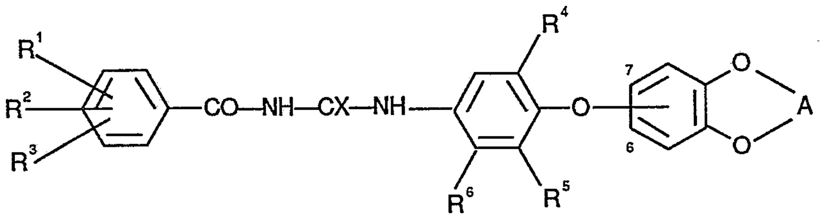

(I)

in which
A represents the $-CF_2-CF_2-$, $-CF_2-CHF-$ or $-CF_2-CFCl-$ group,
X represents oxygen or sulphur,
$R^1$ represents halogen or $C_1-C_4$-alkyl,
$R^2$ and $R^3$ are identical or different and represent hydrogen, halogen or $C_1-C_4$-alkyl,
$R^4$ and $R^5$ are identical or different and represent hydrogen, halogen, $C_1-C_4$-alkyl or $C_1-C_4$-halogenalkyl and
$R^6$ represents hydrogen, characterised in that
a) substituted anilines of the general formula (II)

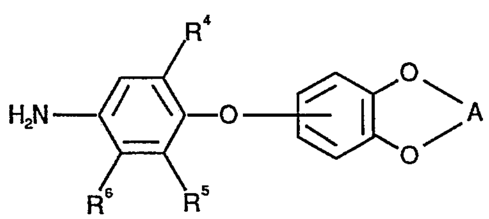

(II)

in which
A, $R^4$, $R^5$ and $R^6$ have the abovementioned meanings, are reacted with benzoyl iso(thio)cyanates of the general formula (III)

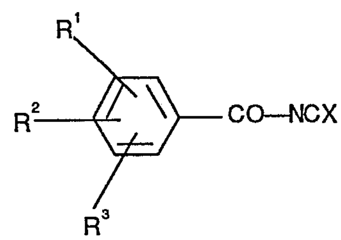

(III)

in which
X, $R^1$, $R^2$ and $R^3$ have the abovementioned meanings, if appropriate in the presence of a diluent, or
b) substituted phenyl iso(thio)cyanates of the general formula (IV)

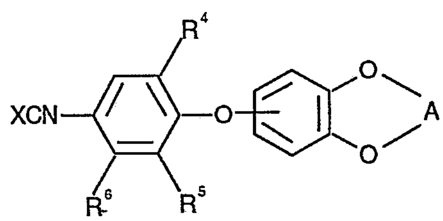

(IV)

in which
A, X, $R^4$, $R^5$ and $R^6$ have the abovementioned meanings, are reacted with benzoic acid amides of the general formula (V)

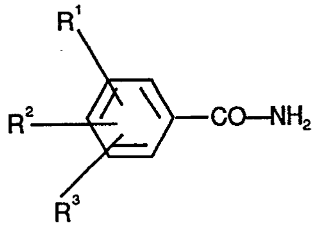
(V)

in which
$R^1$, $R^2$ and $R^3$ have the abovementioned meanings, if appropriate in the presence of a catalyst and if appropriate in the presence of a diluent.

4. Agents for combating pests, characterised in that they contain at least one compound of the formula I according to Claim 1 or 3.

5. Use of compounds of the formula I according to Claim 1 or 3 for combating pests, in particular insects and mites.

6. Method of combating pests, characterised in that compounds of the formula I according to Claim 1 or 3 are allowed to act on the pests, preferably insects or mites, or their environment.

7. Process for the preparation of agents for combating pests, characterised in that compounds of the formula I according to Claim 1 or 3 are mixed with extenders and/or surface-active agents.

8. Compounds of the general formula (XI)

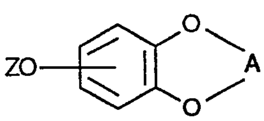
(XI)

in which
A represents the $-CF_2-CF_2-$, $-CF_2-CHF-$ or $-CF_2-CFCl-$ group,
Z represents hydrogen or the radicals

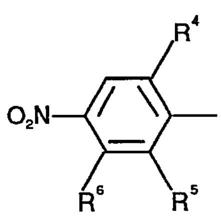
or
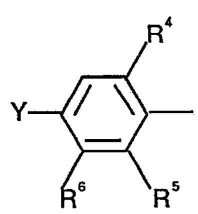

wherein
$R^4$ and $R^5$ are identical or different and represent hydrogen, halogen, $C_1$-$C_4$-alkyl or $C_1$-$C_4$-halogenoalkyl,
$R^6$ represents hydrogen, and
Y represents the $NH_2$ or NCX group,
wherein
X denotes oxygen or sulphur.

9. Process for the preparation of the compounds of the general formula (XI)

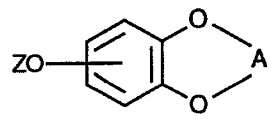
(XI)

in which
A represents the $-CF_2-CF_2-$, $-CF_2-CHF-$ or $-CF_2-CFCl-$ group and
Z represents hydrogen or the radicals

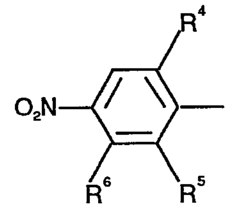
or
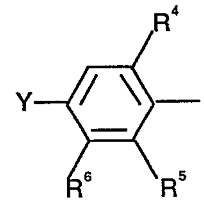

wherein
$R^4$ and $R^5$ are identical or different and represent hydrogen, halogen, $C_1$-$C_4$-alkyl or $C_1$-$C_4$-halogenoalkyl,
$R^6$ represents hydrogen and
Y represents the $NG_2$ or NCX group,
wherein
X denotes oxygen or sulphur, characterised in that compounds of the general formula (X)

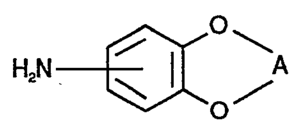
(X)

in which
A has the abovementioned meaning, are diazotised and the diazonium salt formed is decomposed by boiling in acid solution, if appropriate in the presence of a copper salt, and the resulting compounds of the general formula IX

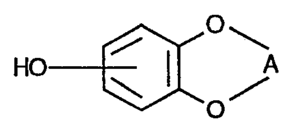
(IX)

in which
A has the abovementioned meaning, are isolated in the customary manner, and, if appropriate (to prepare the compounds of the general formula (VII)), these compounds, if appropriate in the form of their salts or if appropriate in the presence of a base, are reacted with compounds of the general formula (VIII)

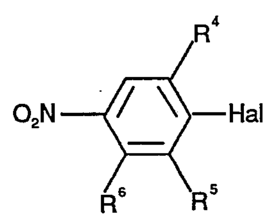
(VIII)

in which
Hal represents halogen, and the resulting compounds of the general formula (VII)

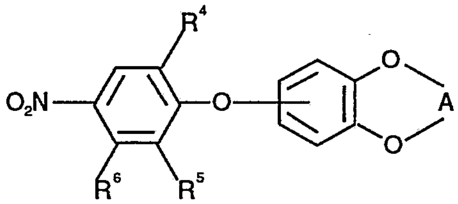

(VII)

in which
A, $R^4$, $R^5$ and $R^6$ have the abovementioned meaning, are isolated in the customary manner and, if appropriate (to prepare the compounds of the general formula (II)), are reduced catalytically or with metals or metal salts, and the resulting compounds of the general formula (II)

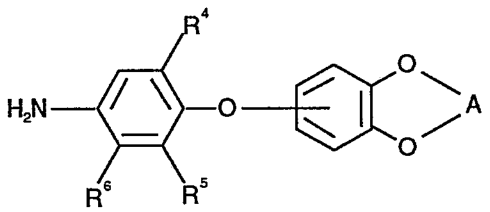

(II)

in which
A, $R^4$, $R^5$ and $R^6$ have the abovementioned meaning, are isolated in the customary manner and, if appropriate (to prepare the compounds of the general formula (IV)), are reacted with phosgene or thiophosgene, and the resulting compounds of the general formula (IV)

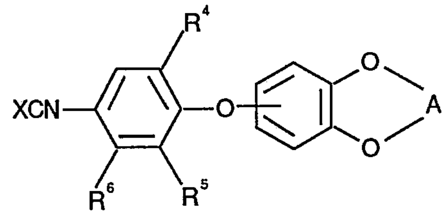

(IV)

in which
A, X, $R^4$, $R^5$ and $R^6$ have the abovementioned meaning, are isolated.

**Revendications**

1. Composés de formule générale (I)

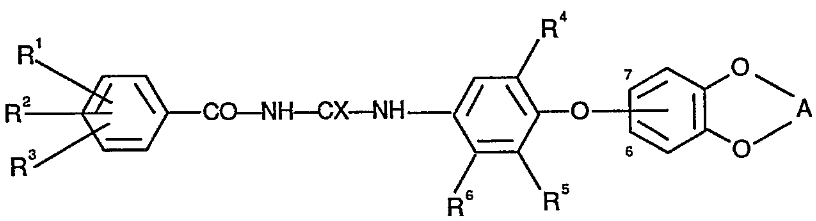

(I)

dans laquelle
A représente les groupes $-CF_2-CF_2-$, $-CF_2-CHF-$ ou $-CF_2-CFCl-$,
X représente l'oxygène ou le soufre,
$R^1$ représente un halogène ou un groupe alkyle en $C_1-C_4$,
$R^2$ et $R^3$, ayant des significations identiques ou différentes, représentent chacun l'hydrogène, un halogène ou un groupe alkyle en $C_1-C_4$,
$R^4$ et $R^5$, ayant des significations identiques ou différentes, représentent chacun l'hydrogène, un halogène, un groupe alkyle en $C_1-C_4$ ou halogénoalkyle en $C_1-C_4$, et
$R^6$ représente l'hydrogène.

2. Composés selon la revendication 1, dans lesquels
A représente les groupes $-CF_2-CF_2-$, ou $-CF_2-CHF-$ ou $-CF_2-CFCl-$,
X représente l'oxygène ou le soufre, de préférence l'oxygène,
$R^1$ représente le fluor, le chlore, le brome ou un groupe méthyle,
$R^2$ et $R^3$, ayant des significations identiques ou différentes, représentent chacun l'hydrogène, le fluor ou le chlore,
$R^4$ et $R^5$, ayant des significations identiques ou différentes, représentent chacun l'hydrogène, le fluor, le chlore, un groupe méthyle ou trifluorométhyle, et
$R^6$ représente l'hydrogène.

3. Procédé de préparation des composés de formule générale (I)

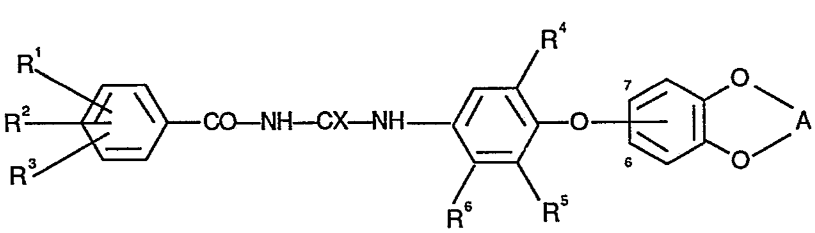

(I)

dans laquelle
A représente les groupes $-CF_2-CF_2-$, $-CF_2-CHF-$ ou $-CF_2-CFCl-$, X représente l'oxygèner ou le soufre,
$R^1$ représente un halogène ou un groupe alkyle en $C_1-C_4$,
$R^2$ et $R^3$, ayant des significations identiques ou différentes, représentent chacun l'hydrogène, un halogène ou un groupe alkyle en $C_1-C_4$,
$R^4$ et $R^5$, ayant des significations identiques ou différentes, représentent chacun l'hydrogène, un halogène, un groupe alkyle en $C_1-C_4$ ou halogénoalkyle en $C_1-C_4$, et
$R^6$ représente l'hydrogène, caractérisé en ce que:
a) on fait réagir des anilines substituées de formule générale (II)

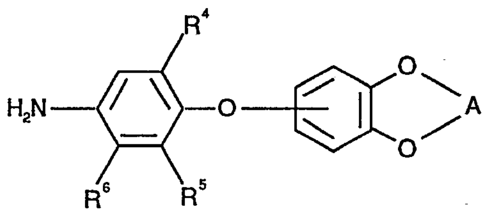

(II)

dans laquelle A, $R^4$, $R^5$ et $R^6$ ont les significations indiquées ci-dessus, avec des benzo-iso(thio)cyanates de formule générale (III)

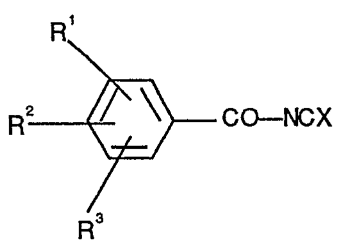

(III)

dans laquelle X, $R^1$, $R^2$ et $R^3$ ont les significations indiquées ci-dessus, éventuellement en présence d'un diluant, ou bien
b) on fait réagir des phényl-iso(thio)cyanates substitués de formule générale (IV)

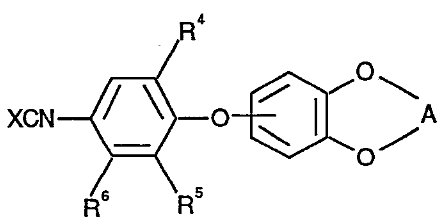

(IV)

dans laquelle A, X, $R^4$, $R^5$ et $R^6$ ont les significations indiquées ci-dessus, avec des benzamides de formule générale (V)

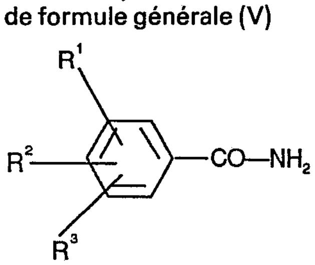

(V)

dans laquelle $R^1$, $R^2$ et $R^3$ ont les significations indiquées ci-dessus, éventuellement en présence d'un catalyseur et éventuellement en présence d'un diluant.

4. Produit pesticide, caractérisé en ce qu'il contient au moins un composé de formule (I) selon les revendications 1 ou 3.

5. Utilisation des composés de formule (I) selon les revendications 1 ou 3 dans la lutte contre les parasites, en particulier les insectes et les acariens.

6. Procédé pour combattre les parasites, caractérisé en ce que l'on fait agir des composés de formule (I) selon les revendications 1 ou 3 sur les parasites, de préférence des insectes ou des acariens, ou leur habitat.

7. Procédé de préparation de produits pesticides, caractérisé en ce que l'on mélange des composés de formule (I) selon les revendications 1 ou 3 avec des diluants et/ou des agents tensioactifs.

8. Composés de formule générale (XI)

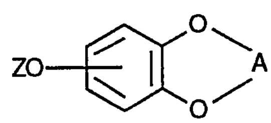

(XI)

dans laquelle
A représente les groupes $-CF_2-CF_2-$, $-CF_2-CHF-$ ou $-CF_2-CFCl-$,
Z représente l'hydrogène ou les groupes

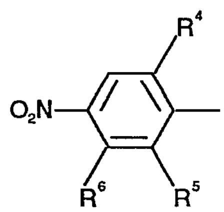

ou

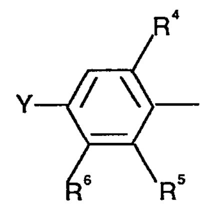

dans lesquels
$R^4$ et $R^5$, ayant des significations identiques ou différentes, représentent chacun l'hydrogène, un halogène, un groupe alkyle en $C_1-C_4$, ou halogénoalkyle en $C_1-C_4$,
$R^6$ représente l'hydrogène, et
Y représente les groupes $NH_2$ ou NCX et
X représente l'oxygène ou le soufre.

9. Procédé de préparation des composés de formule générale (XI)

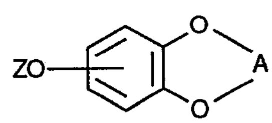

(XI)

dans laquelle
A représente les groupes $-CF_2-CF_2-$, ou $-CF_2-CHF-$ ou $-CF_2-CFCl-$,
Z représente l'hydrogène ou les groupes

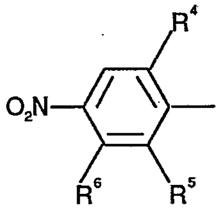

ou

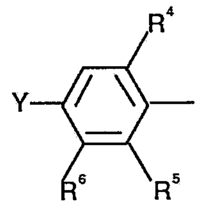

dans lesquels

$R^4$ et $R^5$, ayant des significations identiques ou différentes, représentent chacun l'hydrogène, un halogène, un groupe alkyle en $C_1$–$C_4$ ou halogénoalkyle en $C_1$–$C_4$,

$R^6$ représente l'hydrogène, et

Y représente les groupes $NH_2$ ou NCX, et

X représente l'oxygène ou le soufre,

caractérisé en ce que l'on diazote les composés de formule générale (X)

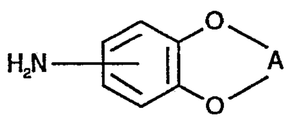

(X)

dans laquelle A a les significations indiquées ci-dessus, on fait bouillir le sel de diazonium formé en solution acide et éventuellement en présence d'un sel de cuivre puis on isole les composés formés répondant à la formule générale (IX)

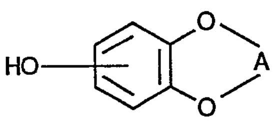

(IX)

dans laquelle A a les significations indiquées ci-dessus, de la manière habituelle, et le cas échéant, pour préparer les composés de formule générale (VII), on fait réagir ces composés, éventuellement à l'état de sels ou éventuellement en présence d'une base, avec des composés de formule générale (VIII)

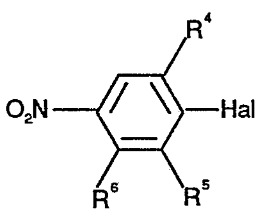

(VIII)

dans laquelle Hal représente un halogène, ce qui donne les composés de formule générale (VII)

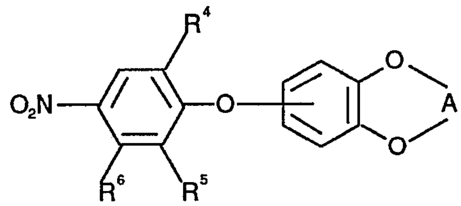

(VII)

dans laquelle A, $R^4$, $R^5$ et $R^6$ ont les significations indiquées ci-dessus, qu'on isole de la manière habituelle et que, le cas échéant (pour la préparation des composés de formule générale (II)), on soumet à réduction catalytique ou à l'aide de métaux ou de sels métalliques, ce qui donne les composés de formule générale (II)

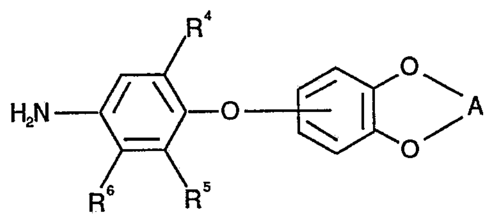

(II)

dans laquelle A, $R^4$, $R^5$ et $R^6$ ont les significations indiquées ci-dessus, qu'on isole de la manière habituelle et que, le cas échéant (pour préparer les composés de formule générale (IV)), on fait réagir avec le phosgène ou le thiophosgène, ce qui donne les composés de formule générale (IV)

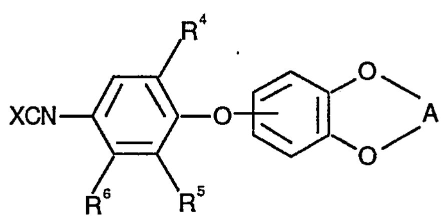

(IV)

dans laquelle A, X, $R^4$, $R^5$ et $R^6$ ont les significations indiquées ci-dessus, qu'on isole.